(19) Europäisches Patentamt
European Patent Office
Office européen des brevets

(11) Numéro de publication: **0 100 015**
**B1**

# (12) FASCICULE DE BREVET EUROPEEN

(45) Date de publication du fascicule du brevet: **02.05.85**

(21) Numéro de dépôt: **83106757.4**

(22) Date de dépôt: **09.07.83**

(51) Int. Cl.⁴: **C 07 C 11/21**, A 23 L 1/226, C 07 C 1/34

**(54) Hydrocarbure aliphatique polyinsaturé, procédé pour sa préparation et son utilisation en tant qu'ingrédient parfumant et aromatisant.**

(30) Priorité: **22.07.82 CH 4469/82**

(43) Date de publication de la demande: **08.02.84 Bulletin 84/6**

(45) Mention de la délivrance du brevet: **02.05.85 Bulletin 85/18**

(84) Etats contractants désignés: **CH DE FR GB LI NL**

(56) Documents cités:
**CH - A - 581 591**

**LIEBIGS ANNALEN DER CHEMIE, 12 mars 1982, pages 579-584, Verlag Chemie GmbH, Weinheim, DE, F.J. MARNER et al.: "Synthese isomerer 1,3,5,8-Undecatetraene"**

(73) Titulaire: **FIRMENICH SA, 1, route des Jeunes, CH-1211 Genève 8 (CH)**

(72) Inventeur: **Escher, Sina Dorothea, Dr., 8, avenue du Lignon, CH-1219 Le Lignon (CH)**
Inventeur: **Morris, Anthony Francis, Route de Trélex, CH-1261 Gingins (CH)**

(74) Mandataire: **Salvadori, Giuseppe, Dr., c/o Firmenich S.A. Case Postale 239, CH-1211 Genève 8 (CH)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 100 015 B1**

## Description

La présente invention a trait au domaine de la parfumerie; elle concerne plus particulièrement l'utilisation d'un hydrocarbure aliphatique polyinsaturé à titre d'ingrédient parfumant. Il s'agit plus précisément du 1,3,5,7-undécatétraène, entité chimique nouvelle, de son utilisation dans le domaine de la parfumerie et des arômes et de sa préparation.

L'invention est définie comme indiqué aux revendications.

Vu la présence de quatre liaisons oléfiniques dans sa molécule, le 1,3,5,7-undécatétraène peut, bien entendu, se présenter sous diverses formes isomériques. Sauf indication particulière, lorsqu'il est fait ici mention dudit composé, l'on entend se référer indifféremment à l'un quelconque de ses isomères ou à tout mélange contenant deux au moins de ceux-ci.

Certaines oléfines polyinsaturées ont été décrites dans l'art antérieur comme possédant des caractères olfactifs intéressants.

P. Teisseire *et al.* dans «Recherches», *16*, 5 (1967) ont fait état de la présence, dans l'huile essentielle de galbanum, de deux isomères d'undécatriène, le (3Z, 5Z)-1,3,5-undécatriène et le (3Z, 5E)-1,3,5-undécatriène.

Le brevet suisse N° 581591 décrit l'emploi de (3E, 5Z)-1,3,5-undécatriène et de son isomère (3E, 5E) en tant qu'ingrédients parfumants. Enfin, F.-J. Marner *et al.* dans «Liebigs Ann. Chem.», *1982*, 579-584 et dans «Naturwissenschaften», *68*, 478-480 (1981) décrivent l'isolation et la synthèse de (3E, 5Z, 8Z)-1,3,5,8-undécatétraène qu'ils reconnaissent comme le principe actif de l'odeur des gamètes des algues brunes, *Spermatochnus paradoxus*. Ce même undécapolyène avait été également identifié par R. E. Moore parmi les composés volatils d'autres algues marines, la *Dictyopteris divaricata*.

Nous avons maintenant découvert que le 1,3,5,7-undécatétraène, sous l'une quelconque de ses formes isomériques, possédait des propriétés odorantes particulièrement utiles et, de ce fait, pouvait être employé avantageusement en tant que modificateur ou renforçateur dans des parfums, compositions parfumantes et produits parfumés ainsi que comme agent aromatisant.

Quoique isomère des composés cités dans l'art antérieur, le composé de l'invention possède des caractères olfactifs et aromatiques originaux et distincts de ceux manifestés par les dérivés cités plus haut.

Il s'est révélé en effet que le 1,3,5-undécatriène possède un caractère odorant vert, gras, huileux, tandis que le composé de l'invention, tout en développant une odeur verte, possède un caractère frais et naturel qui rappelle notamment celui des agrumes, en particulier de leur pelure, tout spécialement du citron et du pamplemousse.

Quoique Marner *et al.* *(op. cit.)* n'aient pas décrit l'odeur du 1,3,5,8-undécatétraène isolé, nous avons constaté que l'odeur de ce dernier était de type herbal, rappelait la mer ou les algues, et ne possédait pas les caractéristiques que l'on a pu retrouver dans le composé de l'invention.

Doté d'une rare puissance olfactive, le composé de l'invention peut servir, même utilisé à de très faibles concentrations, à la reconstitution d'huiles essentielles de nature variée, ainsi qu'à la manufacture de parfums et de bases parfumantes. Grâce à ses propriétés, le 1,3,5,7-undécatétraène peut se combiner avantageusement avec la plupart des ingrédients parfumants courants, aussi bien naturels que synthétiques. A la lueur des indications qui précèdent et des exemples d'utilisation donnés ci-après, l'expert dans l'art pourra aisément imaginer le genre spécifique des coïngrédients pouvant entrer dans une composition donnée avec l'hydrocarbure de l'invention.

Comme souvent en pareil cas, les proportions dans lesquelles le 1,3,5,7-undécatétraène peut développer des notes odorantes utiles varient dans une gamme de valeurs assez étendue. Toutefois, à cause de sa puissance, ces valeurs seront généralement petites. Des proportions de l'ordre de 0,01% en poids par rapport au poids de la composition parfumante ou du produit dans lequel il est incorporé peuvent déjà produire un effet marqué. Bien entendu, ces valeurs peuvent être supérieures à celle indiquée ci-dessus lors de la manufacture de compositions concentrées, des cœurs en particulier.

Lorsque le 1,3,5,7-undécatétraène de l'invention est utilisé dans le domaine des arômes, il sert à développer des notes gustatives de type terreux, boisé. Son goût rappelle celui développé par l'essence de mandarine de variété américaine, notamment par son caractère typique vert boisé. En comparaison avec le 1,3,5,8-undécatétraène décrit dans l'art antérieur, le composé de l'invention présente une note gustative plus florale avec un aspect boisé d'essence d'angélique.

Les proportions préférentielles d'emploi dans un produit terminé, aliment ou boisson par exemple, sont de l'ordre de quelques parties par million en poids.

Le 1,3,5,7-undécatétraène peut être directement ajouté au produit que l'on désire aromatiser au cours de n'importe quelle étape du processus de conditionnement. De préférence, il est cependant utilisé en mélange avec d'autres ingrédients aromatisants usuels, naturels ou synthétiques, des supports, tels que par exemple la gomme arabique ou la dextrine, ou des solvants comestibles, comme l'alcool éthylique, le polyéthylèneglycol ou la triacétine.

Le composé de l'invention est, comme indiqué plus haut, un composé nouveau, lequel peut être obtenu au moyen d'un procédé qui consiste:

A. en l'addition, en présence d'une base forte d'hex-2-énal à un sel quaternaire du phosphore de formule:

$$CH_2{=}CH{-}CH{=}CH{-}CH_2{-}\overset{\oplus}{P}R_3X^{\ominus} \qquad (I)$$

dans laquelle R sert à désigner un reste dérivé d'un hydrocarbure aromatique, aliphatique ou cycloaliphatique et X représente un atome de

chlore, de brome ou d'iode, ou un groupe $ClO_4$ ou $BF_4$, ou

B. en l'addition, en présence d'une base forte, d'octa-2,4-diénal à un sel quaternaire du phosphore de formule:

$$CH_2=CH-CH_2-\overset{\oplus}{P}R_3X^{\ominus} \qquad (II)$$

dans laquelle les symboles R et X ont le sens défini ci-dessus, et

le cas échéant, en la séparation de l'isomère désiré du mélange réactionnel.

Les étapes A ou B définies ci-dessus peuvent être effectuées conformément à des techniques traditionnelles; elles définissent en effet des types de réaction de Wittig (voir à ce sujet H. O. House, «Modern Synthetic Reactions», Benjamin Inc., New York). On pourra donc utiliser comme base forte des agents basiques de nature variée, tels par exemple un hydrure d'un métal alcalin, un alcoolate de métal alcalin comme le méthylate, l'éthylate ou le tert.-butylate de sodium ou potassium.

On peut également utiliser un agent basique mixte, c'est-à-dire le produit résultant de la réaction d'un dérivé alkylé d'un métal alcalin et d'une amine secondaire. Par métal alcalin, on entend un métal tel que le sodium, le potassium, le lithium ou certains de leurs alliages par exemple. Comme amine secondaire, on peut utiliser la diéthylamine, la diisopropylamine, la pipéridine, la diisobutylamine ou l'éthyltert.-butylamine. Comme agent basique, on utilise de préférence le produit de la réaction de butyllithium et de la diisopropylamine. Cependant, nous avons observé que les rendements les meilleurs en produit final étaient obtenus par l'emploi tout simplement de n-butyllithium.

Suivant le procédé de l'invention, la réaction d'addition d'hexénal et d'octadiénal sur les sels de phosphonium de formules (I) et (II), respectivement, peut s'effectuer en présence d'un solvant organique inerte tel l'éther diéthylique, le dioxanne, le tétrahydrofuranne, le diméthoxyéthane, le diméthylformamide ou le diméthylsulfoxyde par exemple. On peut également utiliser un mélange d'au moins deux desdits solvants.

En général, la réaction s'effectue à une température comprise entre 10 et 60° C, de telles valeurs limites ne devant cependant pas être considérées comme absolues. Pour des raisons d'ordre pratique et économique, ladite réaction est conduite de préférence à une température d'environ 10 à 25° C.

Selon une mise en œuvre particulière du procédé de l'invention, la réaction s'effectue à pression ordinaire et sous atmosphère d'un gaz inerte, l'argon ou l'azote. Par l'emploi de réactifs possédant une conformation définie, il est possible, grâce au procédé de l'invention, de faire en sorte de diriger la réaction vers la formation préférentielle et spécifique d'un des isomères du tétraène de l'invention ou, tout au moins, d'un mélange particulier de certains d'entre eux.

En effectuant par exemple la réaction à l'aide de (E)-2-hexénal et du bromure de (2E)-2,4-pentadiényltriphénylphosphonium en présence de n-butyllithium, on obtient un mélange constitué par le (3E, 5Z, 7E)-1,3,5,7-undécatétraène et le (3E, 5E, 7E)-1,3,5,7-undécatétraène.

Lorsque l'on additionne par contre le (2E, 4E)-2,4-octadiénal sur le bromure d'allyltriphénylphosphonium en présence de la même base, on obtient un mélange constitué par le (3Z, 5E, 7E)-1,3,5,7-undécatétraène et le (3E, 5E, 7E)-1,3,5,7-undécatétraène.

En effectuant cette même réaction à l'aide de (2E, 4Z)-2,4-octadiénal sur le bromure d'allyltriphénylphosphonium, on obtient enfin un mélange constitué par les isomères correspondants de structure (3E, 5E, 7Z) et (3Z, 5E, 7Z).

Les mélanges tels qu'obtenus directement par le procédé décrit ci-dessus sont parfaitement adaptés à la plupart des applications envisagées dans le cadre de la présente invention, tant en parfumerie que dans le domaine des arômes. Toutefois, si cela devait se révéler nécessaire, l'on pourra effectuer une séparation des différents constituants des mélanges obtenus à l'aide de chromatographie préparative en phase gazeuse.

L'invention est illustrée par les exemples suivants dans lesquels les températures sont indiquées en degrés centigrades et les abréviations ont le sens usuel dans l'art.

*Exemple 1:*

*Préparation de (3E, 5Z, 7E)-1,3,5,7-undécatétraène et de (3E, 5E, 7E)-1,3,5,7-undécatétraène*

Une suspension de 40,9 g (100 mmol) de bromure de (2E)-2,4-pentadiényltriphénylphosphonium (obtenu suivant «Helv. Chim. Acta», *58*, 1016 (1975) et brevet français N° 74.19580) dans 400 ml de tétrahydrofuranne (THF) anhydre a été refroidie dans un bain de glace, puis 69 ml (100 mmol) d'une solution de n-butyllithium dans l'hexane y ont été ajoutés goutte à goutte et avec agitation. La solution foncée qui en a résulté a été maintenue 1 h sous agitation à température ambiante puis, une fois refroidie, a été introduite graduellement dans une solution refroidie à −78° de (E)-2-hexénal (9,8 g; 100 mmol) dans 100 ml de THF.

Le mélange foncé résultant a été maintenu sous agitation pendant 2 h à −78°, puis il a été versé dans une solution aqueuse glacée d'HCl à 5%. Par extraction au pentane (1×600 ml; 1×500 ml), on obtient une phase organique qui, après les traitements usuels de lavage et neutralisation avec une solution aqueuse 2N de $NaHCO_3$, une solution aqueuse à 10% de bisulfite de sodium et de nouveau une solution aqueuse 2N de bicarbonate de sodium, suivis de séchage sur du sulfate de magnésium anhydre et concentration, a fourni par distillation, en présence de 2-tert.-butyl-4-hydroxyphénol, 7,8 g (53%) d'un produit blanc mi-cristallin: Eb. 95°/14,6×$10^2$ Pa. Ce produit s'est révélé être constitué par un mélange d'undécatétraènes (3E, 5Z, 7E)-1,3,5,7- et (3E, 5E, 7E)-1,3,5,7- dans le rapport pondéral d'environ 55:45.

Une purification ultérieure a été effectuée par

élution sur une colonne d'alumine à l'aide de pentane. Le filtrat résultant a été redistillé à Eb. 90°/ $14{,}6\times10^2$ Pa pour fournir 6,6 g (45%) d'un produit ayant une pureté d'environ 97,5% et un rapport pondéral en lesdits isomères de 52:48.

Les deux isomères constituant le mélange obtenu ont pu être séparés par chromatographie sur colonne capillaire (OV-1) et sur colonne remplie (Apiezon). Les données analytiques observées étaient les suivantes:

*(3E, 5Z, 7E)-1,3,5,7-undécatétraène*

RMN (360 MHz; $CDCl_3$): 0,92 (t, J = 7, 3H); 1,44 (txq, $J_1 = J_2 = 7$, 2H); 2,12 (dxt, $J_1 = J_2 = 7$, 2H); 5,11 (d, J = 11, 1H); 5,23 (d, J = 16, 1H); 5,76 (dxt, $J_1 = 15$, $J_2 = 7$, 1H); 5,91 (dxd, $J_1 = J_2 = 11$, 1H); 6,00 (dxd, $J_1 = J_2 = 11$, 1H); 6,22 (dxd, $J_1 = 15$, $J_2 = 11$, 1H); 6,44 (dxt, $J_1 = 16$, $J_2 = J_3 = 11$, 1H); 6,53 (dxd, $J_1 = 15$, $J_2 = 11$, 1H); 6,68 (dxd, $J_1 = 15$, $J_2 = 11$, 1H) δ ppm;

SM: 148(48, $M^+$); 133(2), 119(23), 105(64), 91(100), 79(32), 65(17), 41(51).

*(3E, 5E, 7E)-1,3,5,7-undécatétraène*

RMN (360 MHz; $CDCl_3$): 0,90 (t, J = 7, 3H); 1,41 (txq, $J_1 = J_2 = 7$, 2H); 2,08 (dxt, $J_1 = J_2 = 7$, 2H); 5,05 (d, J = 10, 1H); 5,18 (d, J = 16, 1H); 5,72 (dxt, $J_1 = 15$, $J_2 = 7$, 1H); 6,1 (dxd, $J_1 = 15$, $J_2 = 11$, 1H); 6,38 (dxt, $J_1 = 16$, $J_2 = J_3 = 10$, 1H) δ ppm;

SM: 148(62, $M^+$); 133(3), 119(30), 105(76), 91(100), 79(33), 65(12), 41(33).

*Exemple 2:*

*Préparation de (3Z, 5E, 7E)-1,3,5,7-undécatétraène et de (3E, 5E, 7E)-1,3,5,7-undécatétraène*

Un mélange contenant les deux isomères d'undécatétraène indiqués a été préparé suivant le procédé décrit à l'exemple 1. Comme produits de départ, on a utilisé:

- 4,06 g (10,6 mmol) de bromure d'allyltriphénylphosphonium,
- 6,9 ml d'une solution 1,54N (10,6 mmol) d'une solution de n-butyllithium dans l'hexane, et
- 1,31 g (10,6 mmol) de (2E, 4E)-2,4-octadiénal [préparé conformément à la méthode décrite dans «J. Org. Chem.», *23*, 1580 (1958)] dans le THF. On a ainsi obtenu 0,6 g (38%) d'un produit qui, après purification par chromatographie sur 25 g d'alumine (éluant: pentane), a donné 0,48 g du mélange isomérique désiré ayant une pureté de 99%. Le rapport respectif de l'isomère (3Z, 5E, 7E) et de l'isomère (3E, 5E, 7E) était de 48,4:51,6. Ces deux composés ont pu être séparés par chromatographie en phase gazeuse en utilisant une colonne capillaire de type UCON HB ou une colonne remplie (SP-1000). Les caractères analytiques de (3Z, 5E, 7E)-1,3,5,7-undécatétraène étaient les suivants:

RMN (360 MHz; $CDCl_3$): 0,91 (t, J = 7, 3H); 1,42 (txq, $J_1 = J_2 = 7$, 2H); 2,09 (dxt, $J_1 = J_2 = 7$, 2H); 5,14 (d, J = 10, 1H); 5,24 (d, J = 16, 1H); 5,75 (dxt, $J_1 = 15$, $J_2 = 7$, 1H); 5,96 (dxd, $J_1 = J_2 = 10$, 1H); 6,02 (dxd, $J_1 = J_2 = 10$, 1H); 6,58 (dxd, $J_1 = 15$, $J_2 = 10$, 1H); 6,82 (dxt, $J_1 = 16$, $J_2 = 10$, 1H) δ ppm;

SM: 148(60, $M^+$); 133(3), 119(27), 105(77), 91(100), 79(33), 65(11), 41(33).

*Exemple 3:*

*Préparation de (3E, 5E, 7Z)-1,3,5,7-undécatétraène et de (3Z, 5E, 7Z)-1,3,5,7-undécatétraène*

Un mélange contenant les deux isomères d'undécatétraène indiqués a été préparé suivant le procédé décrit à l'exemple 1. Comme produits de départ, on a utilisé:

- 5,28 g (13,8 mmol) de bromure d'allyltriphénylphosphonium dans 55 ml d'éther anhydre;
- 8,9 ml d'une solution 1,54N (13,8 mmol) de n-butyllithium dans l'hexane, et
- 1,71 g (13,8 mmol) de (2E, 4Z)-2,4-octadiénal dans 14 ml d'éther anhydre.

Le produit désiré (560 mg) a été obtenu, sous la forme d'une huile, par distillation à 120°/ $14{,}6\times10^2$ Pa (27%) en présence de 2-tert.-butyl-4-méthoxyphénol.

Par une purification ultérieure à l'aide d'une élution sur une colonne d'alumine (éluant: pentane), on a obtenu un filtrat qui, après évaporation et distillation, a fourni 0,450 g du mélange désiré ayant une pureté de 97% et un contenu de l'isomère (3E, 5E, 7Z)- par rapport à l'isomère (3Z, 5E, 7Z)- de 49,5:50,5.

Le (2E, 4Z)-2,4-octadiénal, utilisé comme produit de départ dans le procédé décrit ci-dessus, a été préparé suivant la méthode décrite par F. Näf *et al.*, «Helv. Chim. Acta», *57*, 1309 (1974).

Les deux isomères constituants du mélange obtenu ont pu être séparés à l'aide de chromatographie sur colonne capillaire. Leurs caractères analytiques étaient les suivants:

*(3E, 5E, 7Z)-1,3,5,7-undécatétraène*

RMN (360 MHz; $CDCl_3$): 0,92 (t, J = 7, 3H); 1,42 (txq, $J_1 = J_2 = 7$, 2H); 2,18 (m, 2H); 5,08 (d, J = 11, 1H); 5,22 (d, J = 16, 1H); 5,48 (dxt, $J_1 = 11$, $J_2 = 7$, 1H); 6,06 (dxd, $J_1 = J_2 = 11$, 1H); 6,39 (dxt, $J_1 = 16$, $J_2 = J_3 = 11$, 1H); 6,54 (dxd, $J_1 = 15$, $J_2 = 11$, 1H) δ ppm;

SM: 148(52, $M^+$); 133(2), 119(25), 105(60), 91(100), 79(32), 65(17), 41(51).

*(3Z, 5E, 7Z)-1,3,5,7-undécatétraène*

RMN (360 MHz, $CDCl_3$): 0,92 (t, J = 7, 3H); 1,42 (txq, $J_1 = J_2 = 7$, 2H); 2,18 (m, 2H); 5,15 (d, J = 11, 1H); 5,24 (d, J = 16, 1H); 5,52 (dxt, $J_1 = 11$, $J_2 = 7$, 1H); 5,99 (dxd, $J_1 = J_2 = 11$, 1H); 6,09 (dxd, $J_1 = J_2 = 11$, 1H); 6,65 (dxd, $J_1 = 16$, $J_2 = 11$, 1H); 6,84 (dxt, $J_1 = 16$, $J_2 = 11$, 1H) δ ppm;

SM: 148(67, $M^+$); 133(4), 119(31), 105(75), 91(100), 79(34), 65(12), 41(40).

*Exemple 4:*

Une composition parfumante de base a été préparée en mélangeant les ingrédients suivants (parties en poids):

| | |
|---|---|
| Bergamote synthétique | 300 |
| Essence de citron | 150 |
| Cyclosia® base[1] | 150 |
| Hedione®[2] | 100 |
| Essence de limette dist. | 50 |
| Mayol®[3] | 100 |
| Essence de romarin | 30 |
| 1,3,5,7-Undécatétraène | 20 |
| | 900 |

[1] Firmenich SA; hydroxycitronellal.

[2] Firmenich SA; 2-pentyl-3-oxocyclopentylacétate de méthyle.

[3] Firmenich SA; cis-4-isopropylcyclohexylméthanol.

L'adjonction de 1,3,5,7-undécatétraène confère à la composition un caractère odorant vert, frais et naturel, qui rappelle celui de la pelure du citron ou du pamplemousse; son aspect citron Cologne s'en trouve ainsi rehaussé.

En remplaçant le 1,3,5,7-undécatétraène par l'isomère 1,3,5,8- correspondant de l'état de la technique, notamment le (3E, 5Z, 8Z)-1,3,5,8-undécatétraène et le (3E, 5E, 8Z)-1,3,5,8-undécatétraène, l'on obtient une nouvelle composition dont le caractère odorant se différencie nettement de celui obtenu par l'adjonction de l'hydrocarbure de l'invention. La note citronnée est en effet réduite, tandis que la note herbale devient plus prononcée.

Comme il a été précisé plus haut, le 1,3,5,7-undécatétraène peut se présenter sous différentes formes stéréo-isomères. Les différents composés qui en résultent possèdent des qualités olfactives analogues et, par conséquent, leur champ d'application est tout à fait similaire. Pour des raisons d'ordre pratique et économique, l'on utilise de préférence le mélange des composés tel que directement issu du procédé décrit aux exemples précédents.

*Exemple 5:*

Un mélange de (3E, 5E, 7E)- et (3E, 5Z, 7E)-1,3,5,7-undécatétraènes, tel qu'obtenu par le procédé décrit à l'exemple 1 ci-dessus, a été soumis à l'évaluation d'un groupe d'experts aromaticiens qui devaient se prononcer sur le goût d'une solution aqueuse le contenant à raison de 2,5 ppm.

*Commentaires:* légèrement floral; note boisée rappelant l'essence d'angélique; légèrement poisson; possède la note verte et boisée typique des mandarines de variété américaine.

## Revendications

1. 1,3,5,7-Undécatétraène.

2. Le 1,3,5,7-undécatétraène selon la revendication 1 sous la forme isomérique (3E, 5E, 7E).

3. Le 1,3,5,7-undécatétraène selon la revendication 1 sous la forme isomérique (3E, 5Z, 7E).

4. Le 1,3,5,7-undécatétraène selon la revendication 1 sous la forme isomérique (3E, 5E, 7Z).

5. Le 1,3,5,7-undécatétraène selon la revendication 1 sous la forme isomérique (3Z, 5E, 7Z).

6. Le 1,3,5,7-undécatétraène selon la revendication 1 sous la forme isomérique (3Z, 5E, 7E).

7. Utilisation de 1,3,5,7-undécatétraène selon l'une des revendications 1 à 6 à titre d'ingrédient parfumant ou aromatisant.

8. Composition parfumante ou aromatisante contenant, à titre d'ingrédient actif, un 1,3,5,7-undécatétraène selon l'une des revendications 1 à 6.

9. Aliment ou boisson contenant, à titre d'ingrédient aromatisant, un 1,3,5,7-undécatétraène selon l'une des revendications 1 à 6.

10. Procédé pour la préparation de 1,3,5,7-undécatétraène, caractérisé:

A. en l'addition, en présence d'une base forte d'hex-2-énal, à un sel quaternaire du phosphore de formule:

$$CH_2=CH-CH=CH-CH_2-\overset{\oplus}{P}R_3X^{\ominus} \quad (I)$$

dans laquelle R sert à désigner un reste dérivé d'un hydrocarbure aromatique, aliphatique ou cycloaliphatique et X représente un atome de chlore, de brome ou d'iode, ou un groupe $ClO_4$ ou $BF_4$, ou

B. en l'addition, en présence d'une base forte, d'octa-2,4-diénal à un sel quaternaire du phosphore de formule:

$$CH_2=CH-CH_2-\overset{\oplus}{P}R_3X^{\ominus} \quad (II)$$

dans laquelle les symboles R et X ont le sens défini ci-dessus, et

le cas échéant, en la séparation de l'isomère désiré du mélange réactionnel.

## Patentansprüche

1. 1,3,5,7-Undecatetraen.

2. 1,3,5,7-Undecatetraen gemäss Anspruch 1 in der (3E, 5E, 7E)-Isomer-Konfiguration.

3. 1,3,5,7-Undecatetraen gemäss Anspruch 1 in der (3E, 5Z, 7E)-Isomer-Konfiguration.

4. 1,3,5,7-Undecatetraen gemäss Anspruch 1 in der (3E, 5E, 7Z)-Isomer-Konfiguration.

5. 1,3,5,7-Undecatetraen gemäss Anspruch 1 der (3Z, 5E, 7Z)-Isomer-Konfiguration.

6. 1,3,5,7-Undecatetraen gemäss Anspruch 1 in der (3Z, 5E, 7E)-Isomer-Konfiguration.

7. Verwendung von 1,3,5,7-Undecatetraen gemäss einem der Ansprüche 1 bis 6 als Parfüm- bzw. Aroma-Bestandteil.

8. Parfüm- bzw. Aroma-Komposition, worin 1,3,5,7-Undecatetraen gemäss einem der Ansprüche 1 bis 6 als aktiver Bestandteil enthalten ist.

9. Nahrungsmittel bzw. Getränk, worin 1,3,5,7-Undecatetraen gemäss einem der Ansprüche 1 bis 6 enthalten ist.

10. Verfahren zur Herstellung von 1,3,5,7-Undecatetraen, dadurch gekennzeichnet, dass man

A) Hex-2-enal zu einem quaternären Phosphorsalz der Formel

$$CH_2=CH-CH=CH-CH_2-\overset{\oplus}{P}R_3X^{\ominus} \quad (I)$$

worin R einen aromatischen, aliphatischen

oder cycloaliphatischen Kohlenwasserstoffrest und X ein Chlor-, Brom- oder Iod-Atom, oder eine $ClO_4$- oder $BF_4$-Gruppe bedeutet, in Anwesenheit einer starken Base addiert, oder

B) Octa-2,4-dienal zu einem quaternären Phosphorsalz der Formel

$$CH_2=CH-CH_2-\overset{\oplus}{P}R_3X^{\ominus} \qquad (II)$$

worin die Symbole R und X die oben angegebene Bedeutung haben, in Anwesenheit einer starken Base addiert, und

ggf. das gewünschte Isomer aus der Reaktionsmischung isoliert.

## Claims

1. 1,3,5,7-Undecatetraene.
2. 1,3,5,7-Undecatetraene according to Claim 1 in the (3E, 5E, 7E) isomeric form.
3. 1,3,5,7-Undecatetraene according to Claim 1 in the (3E, 5Z, 7E) isomeric form.
4. 1,3,5,7-Undecatetraene according to Claim 1 in the (3E, 5E, 7Z) isomeric form.
5. 1,3,5,7-Undecatetraene according to Claim 1 in the (3Z, 5E, 7Z) isomeric form.
6. 1,3,5,7-Undecatetraene according to Claim 1 in the (3Z, 5E, 7E) isomeric form.
7. Utilization of 1,3,5,7-undecatetraene according to any of Claims 1 to 6 as perfuming or flavouring agent.
8. Perfuming or flavouring composition containing, as active ingredient, a 1,3,5,7-undecatetraene according to any of Claims 1 to 6.
9. Foodstuff or beverage containing, as flavouring ingredient, a 1,3,5,7-undecatetraene according to any of Claims 1 to 6.
10. Process for the preparation of 1,3,5,7-undecatetraene, characterized in that

(*A*) hex-2-enal is added, in the presence of a strong base, to a phosphorus quaternary salt of formula

$$CH_2=CH-CH=CH-CH_2-\overset{\oplus}{P}R_3X^{\ominus} \qquad (I)$$

wherein R designates a radical derived from an aromatic, aliphatic or cycloaliphatic hydrocarbon and X represents an atom of chlorine, bromine or iodine, or a $ClO_4$ or $BF_4$ radical, or

(*B*) octa-2,4-dienal is added, in the presence of a strong base, to a phosphorus quaternary salt of formula

$$CH_2=CH-CH_2-\overset{\oplus}{P}R_3X^{\ominus} \qquad (II)$$

wherein symbols R and X have the above-defined meaning, and

if required, in the separation of the desired isomer from the reaction mixture.